# EUROPEAN PATENT SPECIFICATION

(11) **EP 2 136 783 B1**
(45) Date of publication and mention of the grant of the patent: **24.04.2013**
(21) Application number: 08719973.3
(22) Date of filing: 13.03.2008
(51) Int. Cl.: A61K 9/08, A61L 31/06, A61L 31/16, A61L 31/14, C08L 71/08, C08B 37/00

(54) **A METHOD OF PROMOTING MUSCLE TISSUE REPAIR**
VERFAHREN ZUR FÖRDERUNG DER REPARATUR VON MUSKELGEWEBE
MÉTHODE FAVORISANT LA RÉPARATION DU TISSU MUSCULAIRE

(30) Priority: 13.03.2007 US 906521 P
(43) Date of publication of application: 30.12.2009
(60) Divisional application: 12152706.3
(73) Proprietor: BIOLINERX LTD., 91450 Jerusalem (IL)
(72) Inventor: LIFSHITZ, Ran, 52293 Ramat-gan (IL); SHMUEL, Tuvia, 42490 Natania (IL)
(74) Representative: Vossius & Partner
(86) International application number: PCT/IL2008/000348
(87) International publication number: WO 2008/111074

(56) References cited:
- WO-A-99/07416
- WO-A-2005/023176
- WO-A-2005/105167
- US-A1- 2003 072 803
- US-A1- 2004 013 733
- US-A1- 2006 024 357
- LANDA ET AL: "Novel injectable alginate scaffold attenuates progressive infarct expansion and preserves left ventricular systolic and diastolic function late after myocardial infarction" JOURNAL OF MOLECULAR AND CELLULAR CARDIOLOGY, XX, XX, vol. 40, no. 6, 1 June 2006 (2006-06-01), page 998, XP005473309 ISSN: 0022-2828
- LEOR ET AL: "Cells, scaffolds, and molecules for myocardial tissue engineering" PHARMACOLOGY AND THERAPEUTICS, ELSEVIER, GB, vol. 105, no. 2, 1 February 2005 (2005-02-01), pages 151-163, XP005614781 ISSN: 0163-7258

## Description

### FIELD AND BACKGROUND OF THE INVENTION

The present invention relates to the embodiments as characterized by the claims. Also described herein are methods and pharmaceutical compositions for promoting tissue repair, and more particularly methods which utilize in-tissue cross-linkable compositions for promoting repair of damaged muscle tissue such as infarcted myocardial tissue.

Cross-linked polymer gel materials are widely utilized in the biomedical industry. For example, polysaccharide gels have been applied in contact lenses, blood contact materials, controlled release formulations, wound dressings, bioadhesives, membranes, superabsorbents, cell encapsulation and immunoisolation materials, and tissue engineering scaffolds (Suggs et al., J. Biomater. Sci. Polym. 9: 653-666, 1998; Aebischer et al., Transplantation 58: 1275-1277, 1994; and Atala et al. (J. Urol. 150: 745-747, 1993).

The potential use of polysaccharide gel materials for treating damaged heart tissue has been intensively researched during the past decade.

The main focus of research has been on utilizing polysaccharide gels for treating the heart tissue following acute myocardial infarction (AMI). AMI typically causes an acute loss of myocardial tissue and an abrupt increase in loading conditions which induces left ventricular (LV) remodeling. The early phase of LV remodeling involves expansion of the infarct zone, which often results in early ventricular rupture or aneurysm formation. Late remodeling encompasses the entire LV and involves time-dependent dilatation, recruitment of border zone myocardium into the scar, distortion of ventricular shape and mural hypertrophy. Consequently, it may lead to progressive deterioration in contractile function, heart failure and eventually death (Sutton and Sharpe, Circulation 101:2981-2988, 2000; Mann, D. L., Circulation 100:999-1008, 1999; and Jugdutt, B. I., Circulation 108:1395-1403, 2003).

Accordingly, cessation or reversal of progressive chamber remodeling is an important aim of heart failure therapy. Clinical attempts to minimize the devastating effects of AMI have thus far failed to effectively repair the irreversible damage inflicted to the heart tissue (Khand et al., Eur. Heart J. 22:153-164, 2001; Jessup and Brozena, S. N. Engl. J. Med. 348:2007-2018, 2003; and Redfield, M. M., N. Engl. J. Med. 347:1442-1444, 2000).

Recently, attempts to implant living cells in damaged myocardium have given hope for repairing the damaged tissue via promoting tissue regeneration (Etzion et al., J. Mol. Cell Cardiol. 33:1321-1330, 2000; Leor et al., Expert Opin. Biol. Ther. 3:1023-39, 2003; and Beltrami et al., Cell; 114:763-776, 2003). This approach has advanced considerably with the development of 3-D biomaterial scaffolds aimed at supporting implantation of donor cells (e.g., cardiac cells or stem cells) in the myocardium. Lately, 3-D biomaterial scaffolds made of polysaccharide gel were successfully implanted onto damaged myocardium with promising results (Leor et al., Circulation 102:56-61, 2000). However, clinical use of such cell seeded 3-D biomaterial scaffolds is limited due to scarcity of suitable donor cells and the high risk involved in major surgery.

While reducing the present invention to practice the present inventor has surprisingly and unexpectedly uncovered that an injectable non cross-linked polymer solution can effectively promote the repair of a damaged heart muscle tissue in rats.

### SUMMARY OF THE INVENTION

The present invention is defined by the claims.

In more detail the present invention relates to a sterile alginate solution being essentially devoid of free multivalent cations for use in the treatment of a damaged muscle tissue, said sterile alginate solution being capable of self-gelling following deposition in or around the muscle tissue. Hence, the present invention also relates to the use of a sterile alginate solution being essentially devoid of free multivalent cations in the manufacture of a medicament for the treatment of a damaged muscle tissue, said sterile alginate solution being capable of self-gelling following deposition in or around the muscle tissue.

The present invention furthermore relates to an article of manufacturing, comprising a sterile alginate solution being essentially devoid of free multivalent cations for promoting the repair, repairing or mechanically supporting the muscle tissue by forming a gel following deposition in or around muscle tissue; and a packaging material identifying said alginate solution for use in treatment of a human subject.

Also described herein is a method of treating a muscle tissue. The method is effected by providing the muscle tissue with an effective amount of a sterile polymer solution being essentially devoid of free multivalent cations and capable of self-gelling following deposition in or around the muscle tissue.

Also described herein is a method of treating a heart infarction. The method is effected by providing a subject in need thereof with an effective amount of a sterile polymer solution being essentially devoid of free multivalent cations and capable of self-gelling following deposition in or around infarcted myocardium tissue.

Also described herein is a method of treating a heart condition. The method is effected by providing a subject in need thereof with an effective amount of a sterile polymer solution being essentially devoid of free multivalent cations and capable of self-gelling following deposition in

or around a damaged myocardium tissue, thereby treating the heart condition.

Also described herein is a method of treating an ischemic muscle tissue. The method is effected by providing a subject in need thereof with an effective amount of a sterile polymer solution being essentially devoid of free multivalent cations and capable of self-gelling following deposition in or around the ischemic muscle tissue, thereby treating the ischemic muscle tissue.

Also described herein is an article of manufacturing which includes a sterile polymer solution being essentially devoid of free multivalent cations and capable of self-gelling following deposition in or around an ischemic muscle tissue and a packaging material identifying the polymer solution for use in treatment of ischemic muscle tissue.

According to a preferred embodiment of the invention described above, the muscle tissue is a myocardium tissue.

The muscle tissue is selected having a concentration of the multivalent cations sufficient to cause the self-gelling.

The polymer solution described herein may be a polysaccharide solution.

In accordance with the invention, the polysaccharide solution is an alginate solution.

According to a preferred embodiment, the alginate solution is a sodium alginate solution.

According to a preferred embodiment, the alginate having an average molecular weight ranging from 10 to 300 kDa.

According to a preferred embodiment, the alginate concentration ranges from 0.1 to 10 % (w/v).

According to a preferred embodiment, the alginate monomer ratio between α-L-guluronic acid and β-D-mannuronic acid ranges between 1:1 and 3:1.

The step of providing the polymer solution described herein to the muscle tissue may be effected via injection or catheterization.

The catheterization may be intra-arterial catheterization.

The effective amount may range between about 0.5 and 5 ml.

According to a preferred embodiment, the polymer solution further comprises at least one therapeutic agent.

According to a more preferred embodiment, the at least one therapeutic agent is selected from the group consisting of a growth factor, a hormone, an anti ischemic drug, an anti-inflammatory drug, an anti-apoptotic drug and an antibiotic drug.

According to a more preferred embodiment, the heart condition is congestive heart failure.

According to a more preferred embodiment, the ischemic muscle tissue is an ischemic myocardium tissue.

According to a more preferred embodiment, the ischemic muscle tissue is an ischemic striated muscle tissue.

The present invention successfully addresses the shortcomings of the presently known configurations also by providing a novel method of treating a damaged muscle tissue by safely administering to the muscle tissue a therapeutically beneficial polymer solution capable of forming a flexible gel following deposition in or around the tissue, thereby providing substantial therapeutic benefits to the damaged muscle tissue.

### DESCRIPTION OF THE PREFERRED EMBODIMENTS

The present invention utilizes novel pharmaceutical compositions to promote repair of damaged tissue such as infarcted myocardial tissue by delivering to the damaged tissue a polymer solution which is sterile and essentially devoid of free multivalent cations yet capable of self-gelling following deposition in or around the tissue.

The principles and operation of the present invention may be better understood with reference to the accompanying descriptions.

U.S. Patent Application Ser. No. 11/229,119 teaches a cross-linked polymer solution capable of maintaining a liquid state in storage and assuming a gel state following deposition within a body tissue. The cross-linked polymer solution can be administered into a body tissue via injection or catheterization. This application provides a partially cross-linked alginate solution and thus requires careful mixing for preparation.

The present inventors unexpectedly uncovered that an alginate solution devoid of exogenous cations is capable of forming a gel scaffold following administration into muscle tissue. As is illustrated in the Examples section hereinbelow administration of a sterile sodium alginate solution which is essentially devoid of free multivalent cations into myocardium tissue following AMI, promoted cellular repair of such tissue and thus effectively reversed the damage caused by the infarction.

Thus, there is also provided herein a method of treating a muscle tissue. The method is effected by providing the muscle tissue with an effective amount of a sterile polymer solution which is essentially devoid of free multivalent cations and yet is capable of self-gelling without addition of exogenous multivalent cations following deposition in or around the muscle tissue. The *in situ* transition of the polymer solution into gel may be effected via crosslinking with extracellular multivalent cations being present in the muscle tissue.

It should be pointed out that in contrast to the cross-linked polymer solution taught by U.S. Patent Application Ser. No. 11/229,119, the polymer solution of the present intention is not partially or wholly cross-linked prior to being deposited in the target tissue

The term "treating" used herein encompasses the complete range of therapeutically positive effects of administrating the polymer solution of the present invention to a muscle tissue, including improving the tissue function, providing mechanical support and promoting tissue healing and repair processes. The term treating further includes reduction of, alleviation of, and relief of diseases or disorders associated with an ischemic muscle tissue. In addition, the term treating includes prevention or postponement of development of diseases or disorders associated with a damaged tissue.

The phrase "muscle tissue" used herein encompasses any mammalian muscle tissue, preferably a human muscle tissue. The phrase further encompasses a smooth muscle tissue, a striated muscle tissue and a cardiac muscle tissue. Striated (skeletal) muscle tissue is under voluntary control. The muscle fibers are syncytial and contain myofibrils, tandem arrays of sarcomeres. Smooth muscle tissue is made up from long tapering cells, generally involuntary and differs from striated muscle in the much higher actin/myosin ratio, the absence of conspicuous sarcomeres and the ability to contract to a much smaller fraction of its resting length. Smooth muscle cells are found particularly in blood vessel walls, surrounding the intestine (especially the gizzard in birds) and in the uterus. Cardiac muscle tissue is a striated but involuntary tissue responsible for the pumping activity of the vertebrate heart. The individual cardiac muscle cells are not fused together into multinucleate structures as they are in striated muscle tissue. As used herein, the phrases "cardiac muscle tissue" and "myocardium tissue" are interchangeable.

Without being bound to theory, gelling of the polymer solution may result from cross linking of the polymer via multivalent cations which are present in or around the muscle tissue. Accordingly, the muscle tissue of the present invention can be any muscle tissue having a concentration of multivalent cations which is sufficient to cause gellation of the polymer solution.

The phrase "polymer solution" used herein refers to a liquid in which a polymer is uniformly dispersed in a solvent such as water.

A suitable polymer, according to the teaching of the present invention, can be any biocompatible, non-immunogenic and, preferably, bio-erodible polymer which can be cross-linked via multivalent cations to form a hydrogel. The polymer can be in a form of a free-base, an acid, a basic, an anion salt, or a monovalent cation salt.

The term "sterile" used herein refers to a solution substantially free of living cells. Sterility of the polymer solution of the present invention can be ascertained using, for example, the following Microbiological Tests in USP 24: Microbial Limit Tests <61>, Sterility Tests <71> and Sterilization and Sterility Assurance of Compendial Articles <12211>.

The phrase "essentially devoid of free multivalent cations" used herein refers to a polymer solution having a concentration of free multivalent ions being less than 0.001% (w/v). It will be appreciated that in the case of sodium alginate trace amounts of calcium and other multivalent cations may be present in commercial preparations (often up to 1.5% on dry weight basis), since such preparations are derived from algae extracts which contain ions. As such, although trace amounts of ions may exist in the polymer compositions utilized by the present invention, such amounts are so low that the present compositions can be considered essentially free of multivalent ions.

The term "gel" used herein refers to a semisolid colloidal suspension of a solid in a liquid. The term "hydrogel" used herein refers to a gel which contains water as the liquid.

The polymer described herein can be, but is not limited to, a polysaccharide such as alginate or chitosan, gellan gum, collagen, carageenan, hyaluronic acid, poly lactic acid, a polyphosphazine and a polyacrylate. The polymer of the present invention is an alginate.

The term "alginate" used herein refers to a polyanionic polysaccharide copolymer derived from sea algae (e.g., *Laminaria hyperborea, L. digitata, Eclonia maxima, Macrocystis pyrifera, Lessonia nigrescens, Ascophyllum codosum, L. japonica, Durvillaea antarctica, and D. potalorum*) and which includes ß-D-mannuronic (M) and α-L-guluronic acid (G) residues in varying proportions.

Preferably, the alginate of the present invention has a monomer ratio between α-L-guluronic acid and ß-D-mannuronic ranging between 1:1 to 3:1 and a molecular weight ranging between 10 to 300 kDa, more preferably between 25 to 50 kDa.

The alginate of the present invention is preferably a soluble alginate salt such as, but not limited to, sodium alginate, potassium alginate, lithium alginate, rubidium alginate and cesium salts of alginic acid, as well as the ammonium salt, and the soluble alginates of an organic base such as mono-, di-, or tri-ethanolamine alginates, aniline alginates, and the like. More preferably, the polymer of the present invention is sodium alginate. Pharmaceutical grade sodium alginate which comply with all the quality and safety requirements of the European and United States of America (USA) pharmacological regulatory authorities, are readily available from several commercial manufacturers such as, for example, Novamatrix FMC Biopolymers (Drammen, Norway). The final concentration of sodium alginate in the polymer solution of the present invention preferably ranges between 0.1 to 10 % (w/v).

Once the polymer of the invention is completely dissolved the solution is sterilized using standard sterilization methods well known in the art such as, but not limited to, autoclaving (e.g., at 120 °C for 30 min), gamma-irradiation or filter sterilization. Preferably the sterilization is effected via filter sterilization using a 0.2 µm membrane.

Optionally, the polymer solution of the invention further includes at least one therapeutic agent. Suitable therapeutic agents which may be included in the polymer solution of the present invention can be, for example, growth factors (e.g., basic fibroblast growth factor; bFGF), vascular endothelial growth factor (VEGF), insulin-like growth factor (IGF), members of the TGF-family, bone morphogenic proteins (BMP), platelet-derived growth factors, angiopoietins, and other factors such as myogenic factors, transcription factors, cytokines, and homeobox gene products, polynucleotides, polypeptides, hormones, anti-inflammatory drugs, anti ischemic drugs, anti-apoptotic drugs or antibiotic drugs.

Advantageously, the therapeutic agent or agents can be chemically linked to the polymer of the invention. Such linkage can be effected via any known chemical bonding approach, preferably a covalent bond. A suitable covalent bond can be, for example, an ester bond (e.g., a carboxylic ester bond, an oxyalkyl carboxylic ester bond, an amide bond, or a thioester bond), a glycosidic bond, a carbonate bond, a carbamate bond, a thiocarbamate bond, a urea bond or a thiourea bond.

The polymer is alginate and the bond to the therapeutic agent may be effected on a mannuronate monomer location such the alginate polymer retains its ability to cross-link via a glucoronate monomer location.

The polymer solution may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the therapeutic compositions or human or veterinary administration. Such notice, for example, may be of labeling approved by the U.S. Food and Drug Administration for medical devices or drugs, or of an approved product insert. Therapeutic compositions may also be prepared, placed in an appropriate container, and labeled for treatment of an indicated condition, as if further detailed above.

Administration of the polymer solution of the present invention to a subject in need thereof can be effected via injection directly into the target muscle tissue or via parenteral administration. For treating AMI, the polymer solution can be administered intra-arterially, preferably intra-coronarily, via a suitable catheter such as a balloon catheter (see, for example, in Knight et al., Circulation 95:2075-2081, 1997).

The polymer solution can be administered on the same day of the AMI during or after initiating reperfusion, preferably about one day after the AMI, preferably about one day after the AMI, preferably about two days after the AMI, preferably about three days after the AMI, preferably about four days after the AMI, preferably about five days after the AMI, preferably about six days after the AMI, preferably about seven days after the AMI.

The phrase "effective amount" used herein refers to an amount effective to provide a significant therapeutic benefit. The amount of a composition to be administered will, of course, be dependent on the subject being treated, the severity of the affliction, the manner of administration, the judgment of the prescribing physician, etc. An effective amount ranges between 0.1 and 10 ml (dwt between 1 and 1000 mg), preferably between 1 and 5 ml (dwt between 10 and 500 mg) of a concentration ranging between 0.1 and 10% (w/v), preferably between 0.2 and 5% (w/v), preferably between 0.3 and 2% (w/v), preferably between 0.5 and 1.5% (w/v), preferably 1% (w/v).

The polymer solution of the present invention can be used to treat a heart infarction by providing a subject in need thereof with an effective amount of the polymer solution in or around the infarcted myocardium.

In addition, the polymer solution of the present invention can be used to treat a heart condition such as congestive heart failure or mitral valve rigurgitation, by providing a subject in need thereof with an effective amount of the polymer solution in or around a damaged myocardium tissue, thereby treating the heart condition.

In addition, the polymer solution of the present invention can be used to treat diseases and conditions associated with an ischemic muscle tissue, such as an ischemic striated muscle tissue, by providing the ischemic muscle tissue with an effective amount of the polymer solution.

Hence, also described herein is a novel method of treating a muscle tissue by providing the muscle tissue with a sterile polymer solution being essentially devoid of free multivalent cations and capable of self-gelling in situ. The treatment provides substantial therapeutic benefits to the muscle tissue safely and effectively.

As used herein, the term "about" denotes ± 10%.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions; illustrate the invention in a non limiting fashion.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

### EXAMPLE 1

### The effect of Sodium Alginate Injected to Rat Infarcted Myocardium Tissue

### Materials and Methods

### Test Solutions:

An aqueous solution of 1% (w/v) sodium alginate (UP-VLVG, Mw = 41 kDa;
NovaMatrix FMC Biopolymers, Drammen, Norway) was filter-sterilized using a 0.2 µm membrane (Millipore) prior to use. A cross-linked alginate solution (BL-1040) was prepared by *thoroughly* mixing equal parts of filter-sterilized aqueous solutions of 2% (w/v) sodium alginate and of 0.6% (w/v) calcium D-gluconate (USP/NF Grade Dr. Paul Lauman lot. 623784).

### Induction of acute myocardial infarction (AMI)

The rat model of AMI was essentially as described by Leor et al. (Circulation 102:III56-61, 2000), Battler et al. (J Mol Cell Cardiol. 33: 1321-133, 2001) and Leor et al. (Circulation 114: I9.4-100, 2006). Briefly, male Sprague-Dawley rats (250-350 g) were anesthetized with a combination of 40 mg/kg ketamine and 10 mg/kg xylazine, intubated and mechanically ventilated. To induce AMI, animal chests were opened by left thoracotomy, their pericardia were removed and the proximal left coronary arteries were permanently occluded by intramural stitches.

### Administration of test solutions

Seven days following AMI, animals were randomized, anesthetized and their chests were opened under sterile conditions. The infarcted myocardial area was visually recognized based on the appearance of surface scar and wall motion akinesis. A single injection (100-150 µL volume) of BL-1040 (positive control), saline (negative control) or 1% sodium alginate was administered into the infarcted myocardial area using a 29-guage needle. Following injection the surgical incision was sutured closed.

### Ecocardiography

Transthoracic echocardiography was performed within 24 hours (baseline echocardiogram) and 30 days following AMI. Echocardiograms were performed using a commercially available echocardiography system (Sonos 5500, Philips, Andover, MA) equipped with 12-MHz phased-array transducer, as described by Leor et al. (Circulation 114:194-100, 2006). Diastolic function was evaluated using pulse-wave Doppler interrogation of mitral inflow performed in a modified four- chamber view. All measurements were averaged for three consecutive cardiac cycles and were performed blinded to the treatment groups.

### Statistical analysis

Echocardiographic measurements were analyzed using paired t-tests. All tests applied were two-tailed, and p value of 5% or less was considered statistically significant.

### Results

As shown in Table 1 hereinbelow, administering 1% sodium alginate solution to rat infracted myocardium resulted in:
1. A significant reduction in the LV systolic area changes as compared with the saline control (49.2% difference between the means, p ≤ 0.01).
2. A significant reduction in the LV diastolic area changes as compared with the saline control (45.8 % difference between the means, p ≤ 0.01).
3. A significant reduction in the LV systolic diameter changes as compared with the saline control (28.4 % difference between the means, p ≤ 0.05).
4. A significant reduction in the LV diastolic diameter changes as compared with the saline control (26.0 % difference between the means, p ≤ 0.05).
5. A significant reduction in the LV mass changes as compared with the saline control (51.5 % difference between the means, p ≤ 0.01).

**Table 1**

| **Differences between 2-D echocardiogram values obtained on day 1 following AMI (prior to administration; baseline) and on day 30 following AMI (% *chan*ge)¹** | | | |
|---|---|---|---|
| | **Sodium Alginate** | **BL-1040 (positive control)** | **Saline (negative control)** |
| **Number of animals** | 12 | 9 | 12 |
| | | | |
| **LV mass (g)** | | | |
| **P value to saline ²** | 0.01 | 0.02 | |
| **Mean** | 30.00 | 33.68 | 61.88 |
| | | | |
| **LVFS (%) ⁷** | | | |
| **P value to saline ²** | 0.16 | 0.31 | |
| **Mean** | -9.75 | -11.55 | -23.08 |
| | | | |
| **FAC (%) ⁸** | | | |
| **P value to saline ²** | 0.13 | 0.32 | |
| **Mean** | -6.24 | -10.03 | -18.75 |
| | | | |
| **LVESD cm ⁴** | | | |
| **P value to saline ²** | 0.04 | 0.01 | |
| **Mean** | 26.66 | 22.56 | 37.24 |
| | | | |
| **LVEDD ³** | | | |
| **P value to saline ²** | 0.02 | 0.02 | |
| **Mean** | 20.52 | 18.83 | 27.73 |
| | | | |
| **LVES area ⁶** | | | |
| **P value to saline ²** | 0.01 | 0.01 | |
| **Mean** | 46.37 | 47.39 | 91.26 |
| | | | |
| **LVED area ⁵** | | | |
| **P value to saline** ² | 0.01 | 0.01 | |
| **Mean** | 35.95 | 40.30 | 66.38 |

| | | | |
|---|---|---|---|
| ¹ % change = [((echo day 30) - (echo day 1)) / (echo day 1)] x 100 ² P value = the estimated probability, using paired t-test, that the difference between the mean values (treatment vs. saline control) can be ascribed to chance alone. ³ LVEDD = LV end diastolic dimension ⁴ LVESD = LV end systolic dimension ⁵ LV EDA = LV end diastolic area ⁶ LV ESA = LV end systolic area ⁷ LV FS = LV fractional shortening - [(LVIDd-LV[Ds)/LVIDd] x 100 ⁸ FAC = Fractional area change | | | |

Hence, the results clearly illustrate that sodium alginate solution administered to animal myocardium tissue following AMI can effectively and substantially reduce damage to the infarcted tissue and effectively improve the heart function.

### EXAMPLE 2

### Intra-coronary Administration of Sodium Alginate into Dog Infarcted Myocardium Tissue

### Material and methods

### Test Solutions:

Test solutions are: saline (negative control), a cross-linked alginate solution (BL-1040; positive control) and sodium alginate solution (1%). The sodium alginate and BL-1040 solutions are prepared as described in Example 1 hereinabove.

### Induction of acute myocardial infarction (AMI)

Two years old (20 - 25 kg) dogs are anesthetized and intubated and respired with room air. Animals are sedated with intravenous oxymorphone hydrochloride (0.22 mg/kg) and diazepam (0.17 mg/kg) and a plane of anesthesia is maintained with 1-2% isofluorane. The procedures are performed with the chest closed and under sterile conditions. To produce the coronary occlusion, a 2.7 French PTCA balloon catheter is advanced introduced over a 0.014 inch wire through a 4JL guiding catheter through a femoral arteriotomy. A 4.0 mm diameter, 15 mm balloon is used. The guiding catheter and positioned in the Left Main coronary artery and the wire, advanced under fluoroscopic guidance, is positioned in the distal Circumflex coronary artery. The balloon catheter is advanced into the coronary artery over the wire and positioned proximal to the first marginal branch. In all instances, the animals are anticoagulated with 750-1000 USP units of intravenous sodium heparin.

### Intra-coronary administration

Once in place, the PTCA balloon is inflated to occlude the coronary artery. Complete occlusion of the coronary artery is confirmed by ST-segment elevation on the electrocardiogram and near absence of LV posterior wall motion assessed by transthoracic 2-dimensional echocardiography. In all instances, the coronary occlusion is maintained for 3 to 5 hours. At the end of the occlusion, the balloon is deflated to allow for coronary reperfusion.

The test solution (2 ml volume) is injected into the animals' Circumflex coronary artery. To simulate the likely clinical setting for use of the test solution, the coronary injection of the test solution is made either on the same day of the AMI (about one hour following initiating reperfusion), or 2 - 5 days following the AMI. Dogs are randomly assigned to receive any of the test solution. All animals are followed for 4 months following the AMI. Angiographic and echocardiographic measurements are made at baseline, prior to inducing the AMI, just prior to administering the test solutions and at two and four months thereafter.

### End Points

1. Prevention or attenuation of progressive LV dilation based on ventriculographic measurements of LV end-systolic and end-diastolic volume.
2. Prevention of progressive deterioration of LV systolic function based on ventriculographic measurement of LV ejection fraction (EF) and echocardiographic measurement of LV fractional area of shortening (FAS).
3. Prevention or attenuation of infarct expansion based on echocardiographic measurements of systolic thickening of the infarcted LV wall.

### Angiographic and Echocardiographic Measurements

All angiographic and echocardiographic measurements are made during cardiac catheterization under general anesthesia and sterile conditions. Left ventriculograms are obtained with the dog placed on its right side and digitally recorded during the injection of 20 ml of contrast material (RENO-M-60 Squibb, Princeton, NJ). Correction for image magnification is made with a radiopaque calibrated grid placed at the level of the LV. LV end-systolic (ESV) and end-diastolic (EDV) volumes are calculated from LV silhouettes using the area-length method (Dodge et al., Am J Cardiol. 1966; 18:10-24, 1966). Left ventricular EF is calculated as the ratio of the difference of EDV and ESV to EDV times 100 (Sabbah et al., Circulation 89:2852-2859, 1994).

All 2-dimensional echocardiographic measurements are made with the dog placed in the right lateral decubitus position. Echocardiographic studies are performed using a General Electric VIVID 7 Dimension ultrasound system with a 3.5 MHz transducer and recorded on a Mitsubishi MD3000 VHS recorder for off-line analysis. A LV short-axis view at mid-papillary muscle level is recorded and used to calculate the percent fractional shortening (FAS; Kono et at. J Am Coll Cardiol 1992;19:1101-1105, 1992), defined as the difference between the end-diastolic area and the end-systolic area divided by the end-diastolic area times 100. Percent systolic wall thickening of the LV anterior wall, posterior wall and inter-ventricular septum are also measured using the short axis view and each calculated as the ratio of wall thickness at end-diastole to wall thickness at end-systole divided by wall thickness at end-diastole times 100. Extrasystolic and post-extrasystolic beats are excluded from all analysis.

### Statistical Analysis

Within group angiographic and echocardiographic data are analyzed using repeated measures analysis of variance (ANOVA) with alpha set at 0.05. If the overall ANOVA is significant, then pairwise comparisons between pre-treatment (PRE) and 2 and 4 months are performed using the Student-Newman- Keuls test. For this test a p-value lower than 0.05 is considered significant. To assess treatment effect, the change (Δ) in each measure from PRE to 2 months and PRE to 4 months post-treatment is calculated for each of the two study arms. To determine whether significant differences in Δ were present between the control groups and the sodium alginate treated group, a t-statistic for two means is used with p < 0.05 considered significant.

## Claims

1. A sterile alginate solution being essentially devoid of free multivalent cations for use in the treatment of a damaged muscle tissue, said sterile alginate solution being capable of self-gelling following deposition in or around the muscle tissue.

2. The solution for use of claim 1, wherein said muscle tissue is treated by promoting the repair, repairing or mechanically supporting the muscle tissue by forming a gel following deposition in or around muscle tissue.

3. The solution for use of claim 1 or 2, wherein said muscle tissue is myocardial muscle tissue.

4. The solution for use of claim 1 or 2, wherein said muscle tissue is ischemic muscle tissue.

5. An article of manufacturing, comprising
(i) a sterile alginate solution being essentially devoid of free multivalent cations for promoting the repair, repairing or mechanically supporting the muscle tissue by forming a gel following deposition in or around muscle tissue; and
(ii) a packaging material identifying said alginate solution for use in treatment of a human subject.

6. The solution for use of claim 4, wherein said ischemic muscle tissue is ischemic myocardial tissue.

7. The solution for use or article of any of claims 1 to 4 or 5, wherein said muscle tissue comprises a concentration of said multivalent cations sufficient to cause gelling of the solution.

8. The solution for use of any of claim 2 to 4 or 5, wherein said solution comprises sodium alginate.

9. The solution for use of claim 1, wherein said alginate has an average molecular weight ranging from 10 to 300 kDa.

10. The solution for use of claim 1, wherein a concentration of said alginate ranges from 0.1 to 10% (w/v).

11. The solution for use of claim 1, wherein a monomer ratio between α-L-guluronic acid and β-D-mannuronic acid in said alginate ranges between 1 : 1 and 3 : 1.

12. The solution for use of claims 1 to 4, wherein said solution is provided to said muscle tissue via injection, parenteral administration or catheterization.

13. The solution for use of claim 12, wherein said catheterization is intra-arterial catheterization.

14. The solution for use of any of claims 1 to 4 or the article of 5, wherein said solution further comprises at least one therapeutic agent.

15. The solution for use of claim 14, wherein said at least one therapeutic agent is selected from the group consisting of a growth factor, a hormone, an anti-ischemic drug, an anti-inflammatory drug, an anti-apoptotic drug and an antibiotic drug.

16. The solution for use of claim 4, wherein said ischemic muscle tissue is associated with a disease or disorder.

17. The solution for use of claim 16, wherein the disease or disorder is a heart condition.

18. The solution for use of claim 4, wherein said ischemic muscle tissue is an ischemic striated muscle tissue.

19. The solution for use of claim 6, wherein the myocardial tissue is infarcted myocardial tissue.

20. The solution for use of claim 1, wherein the solution comprises a concentration of free multivalent cations which is less than 0.001% (w/v).

21. The solution for use of claim 2, wherein said gel is a hydrogel.

22. The solution for use of claims 1 to 4, wherein said alginate has a molecular weight of 25-50 kDa, a monomer ratio between α-L-guluronic acid and β-D-mannuronic acid ranging between 1:1 and 3:1, and has a final concentration in the solution of between 0.1 to 10% (w/v).

23. The solution for use of claim 22, wherein the concentration is about 1%.

24. The solution for use of claim 22 or 23, wherein the alginate is sodium alginate.

## Patentansprüche

1. Sterile Alginatlösung, die im Wesentlichen frei von freien multivalenten Kationen ist, zur Verwendung in der Behandlung eines geschädigten Muskelgewebes, wobei die sterile Alginatlösung fähig ist, nach dem Aufbringen in oder um das Muskelgewebe selbst zu gelieren.

2. Lösung zur Verwendung nach Anspruch 1, wobei das Muskelgewebe durch Förderung der Regeneration, der Regenerierung oder der mechanischen Stützung des Muskelgewebes durch die Bildung eines Gels nach dem Aufbringen in oder um das Muskelgewebe behandelt wird.

3. Lösung zur Verwendung nach Anspruch 1 oder 2, wobei das Muskelgewebe myokardiales Muskelgewebe ist.

4. Lösung zur Verwendung nach Anspruch 1 oder 2, wobei das Muskelgewebe ischämisches Muskelgewebe ist.

5. Erzeugnis, umfassend
(i) eine sterile Alginatlösung, die im Wesentlichen frei von freien multivalenten Kationen ist, zur Förderung der Regeneration, der Regenerierung oder der mechanischen Stützung des Muskelgewebes durch die Bildung eines Gels nach dem Aufbringen in oder um Muskelgewebe; und
(ii) Verpackungsmaterial, das die Alginatlösung zur Verwendung bei der Behandlung eines menschlichen Individuums bestimmt.

6. Lösung zur Verwendung nach Anspruch 4, wobei das ischämische Muskelgewebe ischämisches myokardiales Gewebe ist.

7. Lösung zur Verwendung oder Erzeugnis nach einem der Ansprüche 1 bis 4 oder 5, wobei das Muskelgewebe eine Konzentration der multivalenten Kationen umfasst, die ausreicht, die Gelierung der Lösung auszulösen.

8. Lösung zur Verwendung oder Erzeugnis nach einem der Ansprüche 2 bis 4 oder 5, wobei die Lösung Natriumalginat umfasst.

9. Lösung zur Verwendung nach Anspruch 1, wobei das Alginat ein mittleres Molekulargewicht im Bereich von 10 bis 300 kDa hat.

10. Lösung zur Verwendung nach Anspruch 1, wobei die Konzentration des Alginats im Bereich von 0,1 bis 10% (Gew./Vol.) liegt.

11. Lösung zur Verwendung nach Anspruch 1, wobei das Monomerverhältnis zwischen α-L-Guluronsäure und β-D-Mannuronsäure in dem Alginat zwischen 1:1 und 3:1 liegt.

12. Lösung zur Verwendung nach einem der Ansprüche 1 bis 4, wobei die Lösung an das Muskelgewebe mittels Injektion, parenteraler Verabreichung oder Katheterisierung verabreicht wird.

13. Lösung zur Verwendung nach Anspruch 12, wobei die Katheterisierung intraarterielle Katheterisierung ist.

14. Lösung zur Verwendung nach einem der Ansprüche 1 bis 4 oder Erzeugnis nach Anspruch 5, wobei die Lösung weiterhin mindestens ein therapeutisches Agens umfasst.

15. Lösung zur Verwendung nach Anspruch 14, wobei das mindestens eine therapeutische Agens ausgewählt ist aus der Gruppe bestehend aus einem Wachstumsfaktor, einem Hormon, einem anti-ischämischen Arzneistoff, einem anti-inflammatorischen Arzneistoff, einem anti-apoptotischen Arzneistoff und einem antibiotischen Arzneistoff.

16. Lösung zur Verwendung nach Anspruch 4, wobei das ischämische Muskelgewebe mit einer Krankheit oder Funktionsstörung assoziiert ist.

17. Lösung zur Verwendung nach Anspruch 16, wobei die Krankheit oder Funktionsstörung ein Herzzustand ist.

18. Lösung zur Verwendung nach Anspruch 4, wobei das ischämische Muskelgewebe ein ischämisches gestreiftes Muskelgewebe ist.

19. Lösung zur Verwendung nach Anspruch 6, wobei das myokardiale Gewebe infarziertes myokardiales Gewebe ist.

20. Lösung zur Verwendung nach Anspruch 1, wobei die Lösung eine Konzentration an freien mulivalenten Kationen umfasst, die geringer als 0,001 % (Gew./Vol.) ist.

21. Lösung zur Verwendung nach Anspruch 2, wobei das Gel ein Hydrogel ist.

22. Lösung zur Verwendung nach den Ansprüchen 1 bis 4, wobei das Alginat ein Molekulargewicht von 25-50 kDa, ein Monomerverhältnis zwischen α-L-Guluronsäure und β-D-Mannuronsäure im Bereich zwischen 1:1 und 3:1 und eine Endkonzentration in der Lösung von zwischen 0,1 und 10% (Gew./Vol.) hat.

23. Lösung zur Verwendung nach Anspruch 22, wobei die Konzentration etwa 1% ist.

24. Lösung zur Verwendung nach Anspruch 22 oder 23, wobei das Alginat Natriumalginat ist.

## Revendications

1. Solution d'alginate stérile essentiellement dépourvue de cations multivalents libres destinée à être utilisée dans le traitement d'un tissu musculaire lésé, ladite solution d'alginate stérile étant capable d'auto-gélification après dépôt dans ou autour du tissu musculaire.

2. Solution pour une utilisation selon la revendication 1, dans laquelle ledit tissu musculaire est traité en favorisant la réparation, en réparant ou en supportant mécaniquement le tissu musculaire par formation d'un gel après dépôt dans ou autour du tissu musculaire.

3. Solution pour une utilisation selon les revendications 1 ou 2, dans laquelle ledit tissu musculaire est un tissu musculaire myocardique.

4. Solution pour une utilisation selon les revendications 1 ou 2, dans laquelle ledit tissu musculaire est un tissu musculaire ischémique.

5. Article manufacturé, comprenant
(i) une solution d'alginate stérile essentiellement dépourvue de cations multivalents libres destinée à promouvoir la réparation, réparer ou supporter mécaniquement le tissu musculaire par formation d'un gel après dépôt dans ou autour du tissu musculaire ; et
(ii) un matériau d'emballage identifiant ladite solution d'alginate destinée à être utilisée dans le traitement d'un sujet humain.

6. Solution pour une utilisation selon la revendication 4, dans laquelle ledit tissu musculaire ischémique est un tissu myocardique ischémique.

7. Solution pour une utilisation ou article selon l'une quelconque des revendications 1 à 4 ou 5, dans laquelle ledit tissu musculaire comprend une concentration desdits cations multivalents suffisante pour provoquer la gélification de la solution.

8. Solution à utiliser ou article selon l'une quelconque des revendications 2 à 4 ou 5, dans laquelle ladite solution comprend un alginate de sodium.

9. Solution pour une utilisation selon la revendication 1, dans laquelle ledit alginate a un poids moléculaire moyen allant de 10 à 300 kDa.

10. Solution pour une utilisation selon la revendication 1, dans laquelle une concentration dudit alginate va de 0,1 à 10 % (p/v).

11. Solution pour une utilisation selon la revendication 1, dans laquelle un rapport de monomères entre l'acide α-L-guluronique et l'acide β-D-mannuronique dans ledit alginate est compris entre 1:1 1 et 3:1.

12. Solution pour une utilisation selon les revendications 1 à 4, dans laquelle ladite solution est administrée audit tissu musculaire par injection, administration par voie parentérale ou cathétérisation.

13. Solution pour une utilisation selon la revendication 12, dans laquelle ladite cathétérisation est une cathétérisation intra-artérielle.

14. Solution pour une utilisation selon l'une quelconque des revendications 1 à 4 ou article selon la revendication 5, dans laquelle/lequel ladite solution comprend en outre au moins un agent thérapeutique.

15. Solution pour une utilisation selon la revendication 14, dans laquelle ledit au moins agent thérapeutique est choisi dans le groupe constitué par un facteur de croissance, une hormone, un médicament anti-ischémique, un médicament anti-inflammatoire, un médicament anti-apoptotique et un médicament antibiotique.

16. Solution pour une utilisation selon la revendication 4, dans laquelle ledit tissu musculaire ischémique est associé à une maladie ou à un trouble.

17. Solution pour une utilisation selon la revendication 16, dans laquelle la maladie ou le trouble est une affection cardiaque.

18. Solution pour une utilisation selon la revendication 4, dans laquelle ledit tissu musculaire ischémique est un tissu musculaire strié ischémique.

19. Solution pour une utilisation selon la revendication 6, dans laquelle le tissu myocardique est un tissu myocardique infarci.

20. Solution pour une utilisation selon la revendication 1, dans laquelle la solution comprend une concentration de cations multivalents libres qui est inférieure à 0,001 % (p/v).

21. Solution pour une utilisation selon la revendication 2, dans laquelle ledit gel est un hydrogel.

22. Solution pour une utilisation selon les revendications 1 à 4, dans laquelle ledit alginate a un poids moléculaire de 25-50 kDa, un rapport de monomères entre l'acide α-L-guluronique et l'acide β-D-mannuronique compris entre 1:1 et 3:1, et a une concentration finale dans la solution comprise entre 0,1 et 10 % (p/v).

23. Solution pour une utilisation selon la revendication 22, dans laquelle la concentration est d'environ 1 %.

24. Solution pour une utilisation selon les revendications 22 ou 23, dans laquelle l'alginate est un alginate de sodium.
